# EUROPEAN PATENT SPECIFICATION

(11) **EP 0 903 582 B1**
(45) Date of publication and mention of the grant of the patent: **14.05.2003**
(21) Application number: 98307258.8
(22) Date of filing: 08.09.1998
(51) Int. Cl.: G01N 33/48

(54) **Ligand binding surfaces**
Liganden bindende Oberflächen
Surfaces attachées des ligands

(30) Priority: 09.09.1997 GB 9719140
(43) Date of publication of application: 24.03.1999
(73) Proprietor: Ortho-Clinical Diagnostics, Amersham, Buckinghamshire HP7 OHJ (GB)
(72) Inventor: Dawkes, Adrian Charles, Windsor, Berkshire SL4 3JU (GB); Hipkiss, Jayne Beverly, Shaw, Newbury Berkshire, RG13 2BL (GB); Edgar, Heather Anne, Tring Hertfordshire HP23 4AY (GB); Lowbridge, Dymphna Ann, Little Haywood, Stafford, ST18 0TS (GB)
(74) Representative: Mercer, Christopher Paul

(56) References cited:
- EP-A- 0 427 984
- EP-A- 0 529 775
- US-A- 5 434 087
- KIBONG ET AL: "Efficient Biotinylation of Nitrocellulose Membrane for Immuno-Filtration Capture Assay" J. BIOCHEM. MOL. BIOL., vol. 30, no. 5, 30 September 1997 (1997-09-30), pages 308-314, XP000974053

## Description

The present invention relates to a method for preparing a ligand binding surface. The present invention also relates to a ligand binding surface prepared by the method of the present invention and use of the ligand binding surface in assays.

Passive adsorption of antibodies and other ligand binding entities to a variety of substrates is one of the most widely used immobilisation methods for the generation of functional surfaces for use in assays. The level of functionality generated using this method is variable due to conformational changes to the ligand binding entity on adsorption (Kochwa *et al.*, 1967; Suter and Butler, 1986; Dierks *et al.*, 1986; Butler *et al.*, 1992; and Davies *et al*. 1995). Improvements in the level of functionality of bioactive surfaces have been made using the biotin/avidin linkage. These improvements are well documented (Butler *et al.*, 1992; Davies *et al.*, 1993; Davies *et al.*, 1994.).

Both streptavidin and avidin have a very high binding affinity (10¹⁵ M⁻¹) for biotin. This binding affinity, coupled with the ability of biotin and its derivatives to be incorporated into various biological materials, endows streptavidin biotin systems with great versatility.

Avidin is a glycoprotein found in egg white and the tissues of birds, reptiles and amphibia. Streptavidin is produced by *Streptomyces avidini*. Both these biotin binding proteins exist as tetrameric proteins having four identical subunits.

Biotin, also known as vitamin H or cis-hexahydro-2-oxo-1H-thieno-(3,4)-imidazole-4-pentanoic acid, is an essential vitamin. In mammals, the tissues having the greatest amounts of biotin are the liver, kidneys and pancreas.

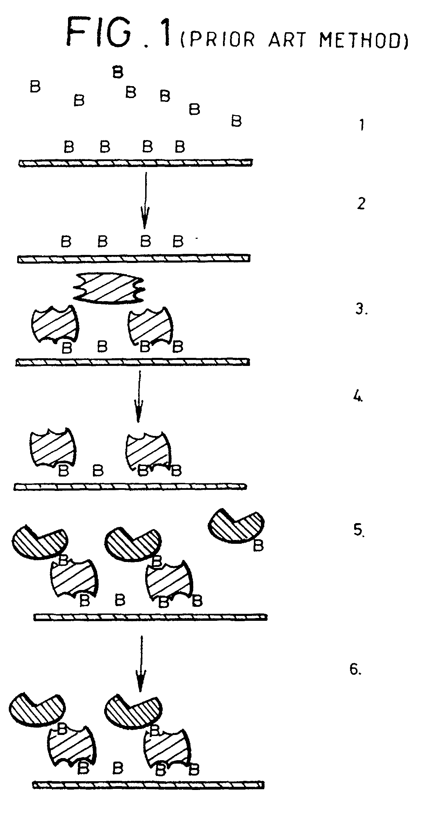

Ligand binding surfaces can be prepared by adding a binding agent (for example, streptavidin or avidin) to a surface having a specific binding partner (for example, biotin) immobilised thereon or coupling the binding agent to the surface, chemically or via a secondary carrier molecule; removing any non-bound components, adding the specific binding partner, to which is attached a ligand receptor (for example, biotinylated ligand receptor) so that complexes comprising binding partner having the ligand receptor attached and binding agent are formed on the surface; and removing any non-bound components. Figure 1 gives a schematic representation of such a method for preparing ligand binding surfaces using biotin as the binding partner and streptavidin as the binding agent.

It can be seen that the preparation of functional surfaces for use in assays is a complex multi-step process. The final step is generally the addition of a binding partner, to which is attached a ligand receptor, in order to produce an analyte specific surface. Control of the quantity of binding partner, to which is attached a ligand receptor, is critical to the performance of the final assay and it is difficult to achieve consistent coating levels, especially where the ligand receptor is required in a limiting concentration, such as in competition immunoassay formats.

There is therefore a need for a method of preparing functional surfaces which can be more easily controlled and which consistently gives functional surfaces having a required coating level. A reduction in the complexity of liquid handling events and improvement in the control of the quantity of ligand receptor coated to the surface are achieved by the present invention. Greater control over the quantity of ligand receptor also improves immunoassay performance in terms of precision, sensitivity and range.

The present invention provides a method for preparing a ligand binding surface comprising:
i. mixing a binding partner, to which is attached a ligand receptor, and a binding agent specific for the binding partner at a specific molar ratio so that complexes are formed which have sufficient free binding sites on the binding agent to allow the complexes to be bound to binding partner immobilised on a surface;
ii. contacting a surface on which binding partner is immobilised with the complexes so that the complexes become attached to the surface; and
iii. removing any unbound components.

The term "binding agent" as used herein means any compound, protein or agent capable of specifically binding to the binding partner. Preferably, the binding agent is SAV or AV, an antibody or antibody fragment such as Fv, Fab and F(ab')₂ fragments, capable of binding to the binding partner. If the binding partner is an antibody or antibody fragment as defined above, then preferably, the binding agent is an antigen or fragment thereof capable of being bound by the binding partner.

In a preferred embodiment of the present invention, the binding agent is any compound, protein or agent that binds biotin. More preferably, the binding agent is an antibody or fragment thereof with affinity for biotin. Most preferably, the binding agent is streptavidin and the functional homolog avidin as well as any recombinant streptavidin or avidin proteins that bind biotin such as those described in WO 97/11183.

The term "binding partner" as used herein means any compound, protein or agent capable of specifically binding to the binding agent. If the binding agent is an antibody or fragment thereof, then preferably, the binding partner is an antigen or fragment thereof capable of binding to the binding agent. If the binding agent is an antigen or fragment thereof, then preferably, the binding partner is an antibody of fragment thereof such as Fv, Fab and F(ab')₂ fragments, capable of binding to the binding agent.

In a preferred embodiment the binding partner is biotin or a derivative or a functional homolog thereof, wherein the binding agent is any compound, protein or agent that binds biotin. Those skilled in the art will be aware that only the intact bicyclic ring of biotin is required for strong binding to streptavidin, avidin or functional homologs thereof. The carboxyl group of biotin's pentanoic acid side chain has little to do with the interaction. Accordingly, biotin derivatives can be prepared by modifying the pentanoic acid carboxyl group without significantly altering the ability of the derivative to bind to streptavidin, avidin or functional homologs thereof. Such biotin derivatives are described in Biomethods, 7, (1996), "A laboratory guide to biotin labelling in biomolecule analysis", T.M. Cier and F. Fahrenholz Eds., Birkhäuser Verlag, Switzerland.

The term "ligand receptor" as used herein means any entity which has the ability to bind to a ligand wherein the ligand may be any desired compound or molecule. Preferred ligand binders include antigens (wherein the ligand is an antibody or antigen binding fragment thereof), antibodies or functional fragments thereof such as Fv, Fab and F(ab')₂ fragments which are capable of binding a ligand, Intrinsic Factor, folate binding protein and cellular receptors.

The surface of the present invention may be any solid support suitable for performing an assay. Preferred surfaces include the surface of a microwell (especially polystyrene microwells), a bead (especially latex/magnetic beads) or a dipstick.

The binding partner can be immobilised on a surface using any of the standard methods known to those skilled in the art. For example, the binding partner can be immobilised on a surface by passive adsorption of the binding partner itself or coupled to a carrier such as bovine serum albumin (BSA), human serum albumin (HSA) or a polyamine such as polylysine. Suitable techniques for immobilising the binding partner are described in Immunoassays, (Diamandis, E.P. and Christopoulos T.K., Eds., Academic Press, London (1996)) especially pages 216 to 222 and 229. Biotinylation of surfaces (i.e. the immobilisation of biotin on to a surface) is described in Bayer and Wilcheck, 1990.

The ligand receptors of the present invention can be attached to the binding partner by any of the standard methods known to those skilled in the art Suitable methods include chemical coupling using cross linking agents and the use of activated binding partners (e.g. biotin active esters) Preferably, the ligand receptors are biotinylated (i.e. attached to biotin) by the method described in Bayer and Wilcheck, 1990. It is further preferred that the ligand receptors are attached to the binding partner in such a way that the ligand receptors' activity is not significantly reduced compared to the free ligand receptors.

In the present invention, the binding agent and binding partner, to which is attached a ligand receptor, are mixed at a specific molar ratio so that complexes are formed which have sufficient free binding sites on the binding agent to allow the complexes to be bound to binding partner immobilised on a surface. Accordingly, the amount of binding agent to be used is governed by the binding capacity of the surface on which binding partner has been immobilised. The amount of the binding partner, to which is attached a ligand receptor, to be complexed with the binding agent is determined by the desired total functionality of the ligand binding surface. This will of course vary depending on the type of assay to be performed (competitive or immunometric) and the ratio of ligand receptors attached to the binding partners.

The present invention allows the binding agent and binding partner, to which is attached a ligand receptor, to be mixed at their final concentrations before they are placed in contact with the surface on which the binding partner is immobilised. This enables greater control over the functionality of the ligand binding surface, greater consistency in the preparation of the ligand binding surface and a subsequent improvement in assay performance.

Preferably, the binding agent and binding partner, to which is attached a ligand receptor, are mixed together so that complexes form. This can take up to 18 hours and is dependent on the concentration of the components used. The surface on which the binding partner is immobilised is contacted with the complexes so that the complexes become attached. This can take up to 16 hours and is dependent on the concentration of free binding sites on the binding agent in the complexes.

In a preferred embodiment of the present invention combinations of binding partners having different ligand receptors attached, are used in preparing the ligand binding surface thereby producing a multi-functional ligand binding surface which can bind more than one ligand. Such multi-functional ligand binding surfaces can be used for several separate assays or for combined assays, especially if different reporter species (for example enzymes, radioactive markers etc) are used for detecting the different bound ligands. Preferably, the present invention provides a method for preparing a multi-functional ligand binding surface comprising biotinylated Intrinsic factor and biotinylated folate binding protein as ligand receptors.

The ligand binding surface of the present invention can be used in a variety of different assays including sandwich assays, competition assays, immunometric assays, assays for antigens and antibodies which are indicative of infectious diseases, and antibody class capture assays. Such assays are described in Immunoassays, (Diamandis, E.P. and Christopoulos T.K., Eds., Academic Press, London (1996)). The ligand binding surface of the present invention may also be used in chromatographic procedures such as affinity chromatography. The ligand binding surface of the present invention may also be used as an immunosensor using, for example, surface plasmon resonance blood bank testing or pregnancy testing.

The present invention is now described further by example with reference to the accompanying figures in which:
Figure 1 shows the prior art method for preparing a ligand binding surface, wherein in step 1, biotinylated carrier (binding partner) is added to the surface; in step 2, excess carrier is washed away; in step 3, streptavidin (binding agent) is added to the surface; in step 4, excess streptavidin is washed away; in step 5, biotinylated ligand receptor (binding partner to which is attached a ligand receptor) is added to the surface; and in step 6, excess biotinylated ligand receptor is washed away;

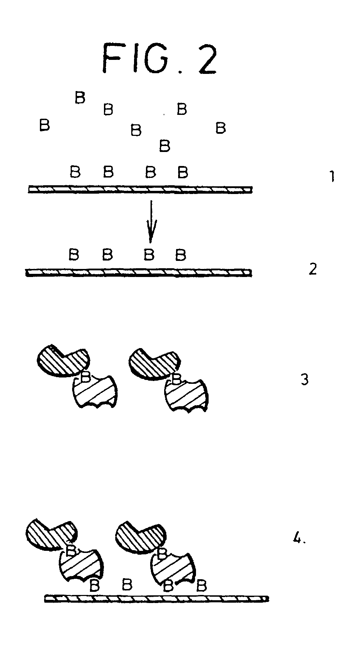

Figure 2 shows the method of the present invention, wherein in step 1, biotinylated carrier (binding partner) is added to the surface; in step 2, excess carrier is washed away; in step 3, streptavidin (binding agent) and biotinylated ligand receptor (binding partner to which is attached a ligand receptor) are mixed together; and in step 4, the streptavidinbiotinylated ligand receptor complex is added to the surface;
Figure 3 is a vitamin B12 standard curve on a dual functional well;
Figure 4 is a folate standard curve on a dual functional well; and
Figure 5 is a block graph showing signal levels obtained using ligand binding surfaces prepared by the method of the present invention and using the prior art method.

### EXAMPLES

### Example 1

### Preparation of a microwell surface for use in a Folate assay.

1. BSA was biotinylated according to published methods (Bayer and Wilchek (1990)), incorporated herein by reference).
2. Polystyrene microwells (obtained from PCMD Kodak) were coated with biotinylated BSA by passive adsorption.
3. Biotinylated Folate binding protein (B-FBP) prepared according to published methods (Bayer and Wilchek (1990)) and streptavidin were mixed in the molar ratio of 1.0x10⁻⁸ moles B-FBP with 1.0x10⁻⁸ moles streptavidin in 0.075M borate buffer pH 9.30. This was left to equilibrate for at least 18 hours at 18-28°C.
4. 200µl of this solution was added to each coated microwell.
5. The microwells were incubated at room temperature for at least 16 hours.
6. The microwells were washed with a Tris buffer solution (pH 8.5, 0.1M) containing 5% sucrose, emptied and dried.

### Folate Assay Protocol

To a test tube add:
1. 150µl of a sample;
2. 50µl stabiliser (0.8% dithiothreitol and 2% ascorbic acid);
3. Mix and incubate at room temperature for 15 min;
4. Add 100µl denaturant (0.85M NaOH, 0.15M disodium tetraborate, 0.01% Mordant Orange dye);
5. Mix and incubate at room temperature for 15 min;
6. Add 100µl neutraliser (0.6M boric acid, 30mM potassium iodate, 2.5% Triton X-100, 0.01% Medical antifoam C (obtained from Dow Corning France));
7. Mix;
8. Transfer 80µl to the coated well;
9. Add 100µl Folate-horseradish peroxidase conjugate reagent, prepared by EDAC coupling (50mM sodium phosphate buffer pH 6.4 containing: folate-HRP conjugate to give 0.16µg/ml HRP concentration; 0.5% human serum albumin; 0.5% bronidox-L; 0.29% sodium chloride; 0.01% potassium ferricyanide; and 5% sucrose);
10. Incubate for 45 min at 37°C with shaking;
11. Wash and add signal reagent using Vitros Eci reagents supplied commercially from Ortho Clinical Diagnostics (O.C.D) Amersham UK; and
12. Measure luminescence after 5 min and before 20 min.

Please note, all percentages are with respect to volume and all solutions are aqueous.

### Folate Assay Results

### Calibration curve for a folate assay using the method of the present invention.

| Calibration Level (Folate ng/ml) | Light Units for Replicate 1 | Light Units Replicate 2 | Mean Light Units |
|---|---|---|---|
| 0 | 60.87 | 61.12 | 61.00 |
| 0.93 | 55.93 | 52.64 | 54.29 |
| 1.83 | 42.22 | 44.87 | 43.55 |
| 4.12 | 23.37 | 22.23 | 22.80 |
| 11.1 | 7.16 | 7.50 | 7.33 |
| 19.4 | 3.03 | 3.00 | 3.02 |

### Calibration curve for folate assay using the prior art method.

| Calibration Level (Folate ng/ml) | Light Units for Replicate 1 | Light Units Replicate 2 | Mean Light Units |
|---|---|---|---|
| 0 | 110.65 | 113.65 | 112.15 |
| 0.93 | 107.65 | 105.22 | 106.44 |
| 1.83 | 91.29 | 91.21 | 91.25 |
| 4.12 | 63.60 | 64.42 | 64.01 |
| 11.1 | 20.93 | 21.43 | 21.18 |
| 19.4 | 6.53 | 6.72 | 6.63 |

Precision for plasma pool containing 3 ng/ml folate (n = 12 replicates) (i.e. the measurement of reproducibility of the folate assay by repeatedly measuring the concentration of one sample (in this case a plasma sample containing 3ng/ml folate)).

### Result using the Method of the Present Invention

| Mean light units | %C.V of light units | Mean Folate dose | %c.v of dose |
|---|---|---|---|
| 20.02 | 2.56 | 3.06ng/ml | 3.25 |

### Results using the Prior art Method

| Mean light units | %C.V of light units | Mean Folate dose | %c.v of dose |
|---|---|---|---|
| 55.02 | 4.59 | 2.92ng/ml | 10.65 |

Assay precision is significantly better using the method of the present invention.

### Example 2

### Preparation of microwell surfaces for use in a Vitamin B12 assay

1. Polystyrene microwells were coated with biotinylated BSA as in Example 1.
2. Biotinylated Intrinsic Factor (B-IF) and streptavidin were mixed at molar ratios of 7 x 10⁻¹⁰ moles B-IF with 14 x 10⁻¹⁰ moles streptavidin in 0.1M phosphate buffer pH 7.0. This was left to equilibrate for at least 18 hours at 20-28°C.
3. 28 x 10⁻¹⁰ moles of biotinylated BSA (B-BSA) was added to bind to BIF-streptavidin complex. A ratio of 1:2:4 of B-IF:streptavidin: B-BSA is required to ensure all IF is bound.
4. 210µl of this solution was added to each coated microwell.
5. The microwells were incubated at room temperature for at least 16 hours.
6. The microwells were washed twice with a Tris buffer and once with a Tris buffer containing 5% sucrose, emptied and dried.

### Vitamin B12 Assay Protocol

To a test tube add:
1. 150µl of a sample;
2. 50µL stabiliser (1.6% Dithiothreitol in 0.8% sodium chloride);
3. 100µl Denaturant (0.85M NaOH, 0.01% KCN, 0.15M disodium tetraborate, 0.01% Mordant Orange dye);
4. Mix and incubate at room temperature for 15 min;
5. Add 100µl neutraliser (0.6M Boric acid, 30 mM potassium iodate, 2.5% Triton X-100, 0.01% Medical antifoam C);
6. Mix;
7. Transfer 60µl to the coated microwell;
8. Incubate for 15 min at 37°C;
9. Add 100µl B-12-horseradish peroxidase conjugate, prepared by EDAC coupling (50mM sodium phosphate buffer pH 6.4 containing: folate-HRP conjugate to give 0.16µg/ml HRP concentration; 0.5% human serum albumin; *0.5%* bronidox-L; 0.29% sodium chloride; 0.01% potassium ferricyanide; and 5% sucrose);
10. Incubate for 30 min at 37°C with shaking;
11. Wash, add signal reagent using reagents as described in Example 1; and
12. Measure luminescence after 5 min and before 20 min.

### Vitamin B12 Assay Results

| Sample Number | Expected Vitamin B12 pg/ml (By RIA) | Vitamin B12 pg/ml prior art method | Vitamin B12 pg/ml Method of invention |
|---|---|---|---|
| 3153 | 149 | 148 | 189 |
| 2993 | 134 | 174 | 131 |
| 2986 | 403 | 386 | 400 |
| 3019 | 204 | 294 | 254 |
| 3133 | 1644 | 857 | 1161 |
| 3230 | 1114 | 749 | 1169 |

Assays performed on microwell surfaces coated using the method of the present invention agree more closely with the expected values from the RIA method and the assay dynamic range was increased.

### Example 3

### Preparation of a dual functional microwell for use in simultaneous or separate B12 & Folate assays

1. Polystyrene microwells were coated with biotinylated carrier (in this case biotinylated BSA).
2. Biotinylated Folate binding protein (B-FBP), biotinylated Intrinsic Factor (B-IF) and streptavidin were mixed in the molar ratios of 1.0 x 10⁻⁸ moles B-FBP with 6.0 x 10⁻¹⁰ moles B-IF and 2.0 x 10⁻⁸ moles streptavidin in 0.075M borate buffer, pH 9.30. This was left to equilibrate for at least 18 hours at 18-20°C.
3. 200µl of this solution was added to each coated microwell.
4. The microwells were incubated at room temperature for at least 16 hours.
5. The microwells were washed with a Tris buffer solution containing 5% sucrose, emptied and dried.

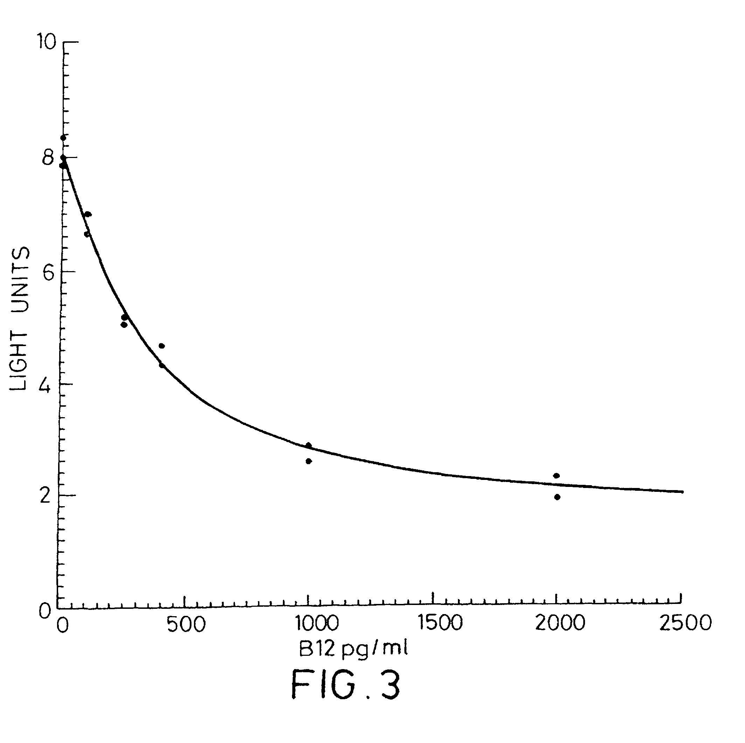

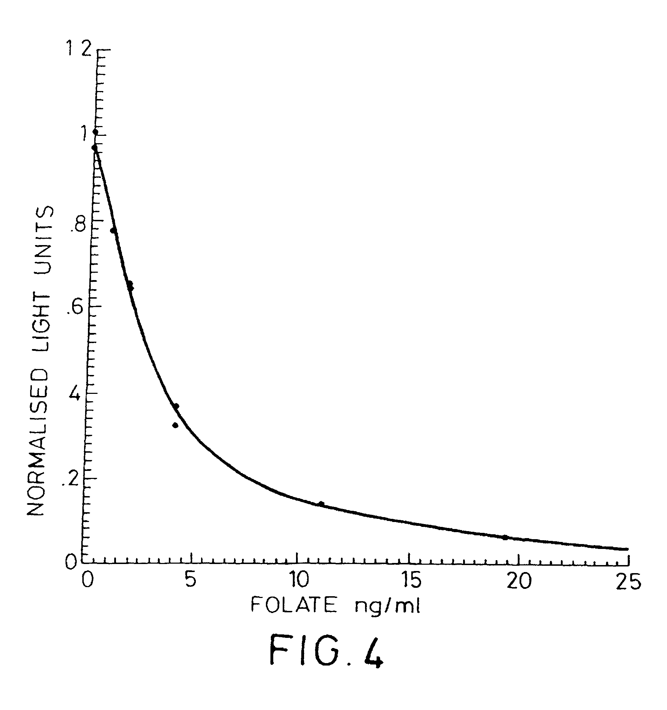

Vitamin B12 and Folate assay protocols are as described in Examples 1 and 2. Standard curves produced using the microwells are shown in Figures 3 and 4.

### Example 4

### Preparation of a microwell for use in an NTx (cross-linked N-telopeptides of Type 1 collagen Assay

1. Polystyrene microwells were coated with biotinylated carrier (in this case biotinylated BSA).
2. Biotinylated NTx peptide and streptavidin were mixed in the molar ratios of 5.13 x 10⁻⁸ moles of peptide of 1.33 x 10⁻⁸ moles streptavidin or 1.00 x 10⁻⁷ moles of peptide to 1.66 x 10⁻⁸ moles of streptavidin in 0.1M sodium phosphate buffer pH 7.0.
3. 200µl of this solution was added to each coated microwell.
4. The microwells were incubated at room temperature for at least 16 hours.
5. The microwells were washed with a Tris buffer (pH 8.4,0.1M) containing 5% sucrose, emptied and dried.

### Evaluation of NTx Wells

1. Add 200µl of a horseradish peroxidase labelled anti-NTx antibody conjugate solution to wells prepared as described above and to wells produced using the prior art method. (The prior art coating method uses biotinylated NTx peptide at 100ng/ml and free biotin at 100ng/ml as the biotinylated ligand binder solution that is added to the wells in step 5 of the prior art coating method shown in Figure 1).
2. Incubate for 30 min at 37°C with shaking;
3. Wash, add signal reagent using Vitros Eci reagents supplied commercially from O.C.D. Amersham UK; and
4. Measure luminescence after 5 min.

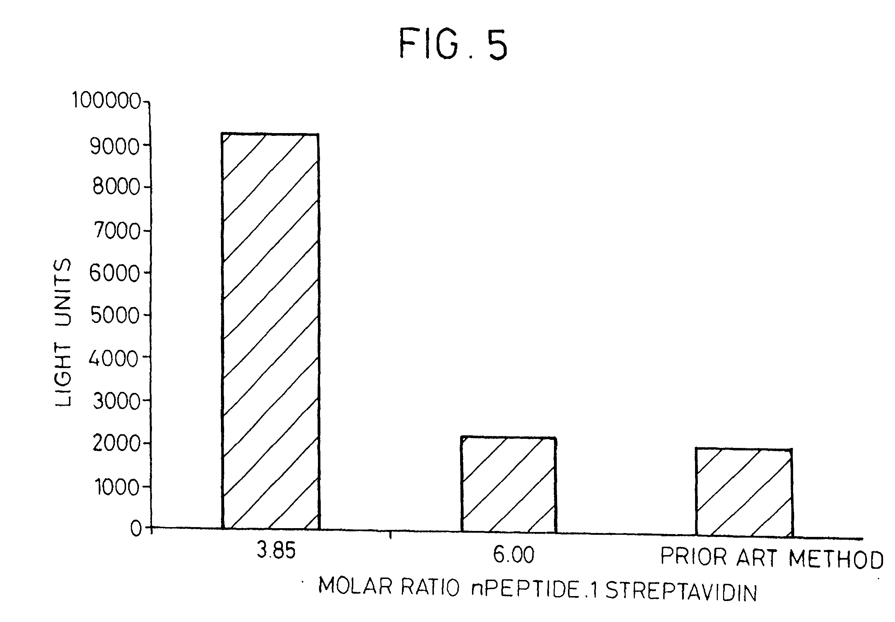

The results are given in Figure 5 and show that the method of the present invention gives a ligand binding surface that has a greater ability to bind ligand.

### References

1. Kochwa, S., Bronwell, M., Rosenfield, R.E. and Wasserman, L.R. (1967) Adsorption of proteins by polystyrene particles. L Molecular unfolding and acquired immunogenicity of IgG. J. Immunol 99.981.
2. Suter, M. and Butler, J.E. (1986) The immunochemistry of sandwich ELISAs. II. A novel system prevents denaturation of capture antibodies. Immunol. Lett. 14 313.
3. Dierks S.E., Butler, J.E. and Richardson, H.B. (1986) Altered recognition of surface-absorbed compared to antigen bound antibodies in the ELISA. Mol. Immunol. 23.927.
4. Butler, J.E., Ni, L., Nessler, R., Joshi, K.S., Suter, M., Rosenberg, B., Chang, J., Brown, W.R. and Cantarero, L.A. (1992) The physical and functional behaviour of capture antibodies adsorbed on polystyrene. J. Immunol. Methods 150, 77-90.
5. Davies, J., Dawkes, A.C., Haymes, A.G., Sunderland, R.F. and Edwards, J.C. (1995) Scanning tunnelling microscopy and dynamic contact angle studies of the effects of partial denaturation on immunoassay solid phase antibody. J. Immunol. Methods 186 111-123.
6. Davies, J., Dawkes, A.G., Haymes, A.G., Roberts, C.J. Sunderland, R.F., Wilkins, MJ., Davies, M.c., Tendler, S.J.B., and Edwards, J.C. (1994), A scanning tunnelling microscopy comparison of passive antibody adsorption and biotinylated antibody linkage to streptavidin on microtitre wells J Immunol. Methods, 167, 263-269.
7. Davies J., Roberts, C.J. Dawkes. A.C., Sefton, J., Edwards, J.C., Claseby, T.O., Haymes, A.G., Davies, M.C., Jackson D.E., Lomas, M., Shakeshift, K.M., Tendler, S.J.B., Wilkins, M.J. and Williams, P.M. (1994) Use of scanning probe microscopy and surface plasmon resonance as analytical tools in the study of antibody coated microtitre wells. Langmuir 10(8) 2654-2661.
8. Bayer, E.A. and Wilchek, M. (1990) Protein biotinylation. In: M Wilchek and E.A. Bayer (Eds.), Methods in Enzymology, Vol. 184. Academic Press, New York, pp 138.

## Claims

1. A method for preparing a ligand binding surface comprising:
i. mixing a binding partner, to which is attached a ligand receptor, and a binding agent specific for the binding partner at a specific molar ratio so that complexes are formed which have sufficient free binding sites on the binding agent to allow the complexes to be bound to binding partner immobilised on a surface;
ii. contacting a surface on which binding partner is immobilised with the complexes so that the complexes become attached to the surface; and
iii. removing any unbound components.

2. The method of claim 1 wherein the binding agent is a compound, protein or agent that binds biotin.

3. The method of claim 2 wherein the binding agent is streptavidin.

4. The method of any one of claims 1 to 3 wherein the binding partner is biotin or a derivative or a functional homolog thereof.

5. The method of any one of the previous claims wherein the ligand receptors are antibodies or functional fragments thereof.

6. The method of any one of claims 1 to 4 wherein the ligand receptors are Intrinsic Factor or folate binding protein.

7. The method of any one of the previous claims wherein the surface is the surface of a microwell, a bead or a dipstick.

8. The method of any one of the previous claims wherein a multi-functional ligand binding surface is prepared using at least two different ligand receptors.

9. The method of claim 8 wherein the different ligand receptors are Intrinsic Factor and folate binding protein.

## Patentansprüche

1. Verfahren zur Herstellung einer Liganden-bindenden Oberfläche, umfassend:
i. Mischen eines Bindungspartners, an den ein Ligandenrezeptor angebracht ist, und eines Bindungsmittels, das für den Bindungspartner spezifisch ist, in einem bestimmten molaren Verhältnis, so daß Komplexe gebildet werden, die ausreichend freie Bindungsstellen auf dem Bindungsmittel aufweisen, um es den Komplexen zu erlauben, an Oberflächen-immobilisierte Bindungspartner zu binden;
ii. In-Kontakt-bringen einer Oberfläche, auf die ein Bindungspartner immobilisiert ist mit den Komplexen, so daß die Komplexe auf die Oberfläche angebracht werden; und
iii. Entfernen jeglicher nicht gebundener Komponenten.

2. Verfahren nach Anspruch 1, wobei das Bindungsmittel eine Verbindung, Protein oder Mittel ist, das Biotin bindet.

3. Verfahren nach Anspruch 2, wobei das Bindungsmittel Streptavidin ist.

4. Verfahren nach einem der Ansprüche 1 bis 3, wobei der Bindungspartner Biotin oder ein Derivat oder ein funktionelles Homolog davon ist.

5. Verfahren nach einem der voranstehenden Ansprüche, wobei die Ligandenrezeptoren Antikörper oder funktionelle Fragmente davon sind.

6. Verfahren nach einem der Ansprüche 1 bis 4, wobei die Ligandenrezeptoren Intrinsischer Faktor oder Folat-bindendes Protein sind.

7. Verfahren nach einem der voranstehenden Ansprüche, wobei die Oberfläche die eines Mikrotüpfels, eines Beads oder eines Meßstabes ist.

8. Verfahren nach einem der voranstehenden Ansprüche, wobei eine multi-funktionelle Liganden-bindende Oberfläche, unter der Verwendung von mindestens zwei verschiedenen Ligandenrezeptoren präpariert wird.

9. Verfahren nach Anspruch 8, wobei die verschiedenen Ligandenrezeptoren Intrinsischer Faktor und Folat-bindendes Protein sind.

## Revendications

1. Procédé pour la préparation d'une surface se liant à un ligand, comprenant :
I. le mélange d'un partenaire de liaison, auquel est fixé un récepteur de ligand, et d'un agent de liaison spécifique du partenaire de liaison, en un rapport molaire particulier, de manière que soient formés des complexes comportant suffisamment de sites de liaison libres sur l'agent de liaison pour permettre aux complexes de se lier au partenaire de liaison immobilisé sur une surface ;
II. la mise en contact d'une surface sur laquelle est immobilisé le partenaire de liaison avec les complexes, de manière que les complexes se fixent à la surface ; et
III. l'élimination de tous composants non fixés.

2. Procédé de la revendication 1, dans lequel l'agent de liaison est un composé, une protéine ou un agent qui fixe la biotine.

3. Procédé de la revendication 2, dans lequel l'agent de liaison est la streptavidine.

4. Procédé de l'une quelconque des revendications 1 à 3, dans lequel le partenaire de liaison est la biotine ou un dérivé ou un homologue fonctionnel de celle-ci.

5. Procédé de l'une quelconque des revendications précédentes, dans lequel les récepteurs de ligands sont des anticorps ou des fragments fonctionnels de ceux-ci.

6. Procédé de l'une quelconque des revendications 1 à 4, dans lequel les récepteurs de ligands sont le Facteur Intrinsèque ou la protéine de liaison au folate.

7. Procédé de l'une quelconque des revendications précédentes, dans lequel la surface est la surface d'un micropuits, d'une bille ou d'une pointe.

8. Procédé de l'une quelconque des revendications précédentes, dans lequel on prépare une surface de fixation de ligand multifonctionnelle en utilisant au moins deux différents récepteurs de ligands.

9. Procédé de la revendication 8, dans lequel les différents récepteurs de ligands sont le Facteur Intrinsèque et la protéine de liaison au folate.
